(19) Europäisches Patentamt
European Patent Office
Office européen des brevets

(11) EP 2 301 415 B1

(12) EUROPEAN PATENT SPECIFICATION

(45) Date of publication and mention
of the grant of the patent:
**23.12.2020 Bulletin 2020/52**

(51) Int Cl.:
***A61B 1/06*** *(2006.01)*

(21) Application number: **10178411.4**

(22) Date of filing: **22.09.2010**

(54) **Endoscope for simultaneous white light and narrow band observation**

Endoskop zur simultanen schmalbandigen und Weisslichtbeobachtung

Endoscope pour l'observation simultanée à la lumière blanche et en bande étroite

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **24.09.2009 JP 2009219242
25.05.2010 JP 2010119748
20.07.2010 JP 2010163442**

(43) Date of publication of application:
**30.03.2011 Bulletin 2011/13**

(73) Proprietor: **Fujifilm Corporation
Minato-ku
Tokyo (JP)**

(72) Inventors:
• **Ozawa, Satoshi**
**Kanagawa (JP)**
• **Iida, Takayuki**
**Kanagawa (JP)**
• **Erikawa, Akihiko**
**Kanagawa (JP)**
• **Endo, Azuchi**
**Kanagawa (JP)**

(74) Representative: **Klunker IP
Patentanwälte PartG mbB
Destouchesstraße 68
80796 München (DE)**

(56) References cited:
**EP-A1- 1 795 798      EP-A1- 1 880 657
EP-A1- 2 052 673      US-A- 5 512 940
US-A1- 2003 176 768   US-A1- 2007 203 413**

**Description**

BACKGROUND OF THE INVENTION

1. Technical Field

The invention relates to an endoscope.

2. Related Art

In recent years, an endoscope has been utilized to perform a so-called special light observation operation of emitting light in a specific narrow wavelength band (narrow-band light) to a mucosal tissue of a body and acquiring tissue information at a desired depth of the body tissue (JP 2002-34893 A (corresponding to US 2003/0176768 A, US 2008/0281154 A and US 2008/0294105 A)). This type of endoscope can simply visualize body information that cannot be obtained from a normal observation image, such as the emphasis of the microscopic structure of the blood vessel which is newly generated in the mucosal layer or the submucosal layer or a site of lesion. For example, in the case where an observation object is a site of cancer lesion, when blue narrow-band light is applied to the mucosal tissue, the microscopic vessel or the microscopic structure in the superficial layer of the tissue can be observer in more detail. Therefore, it is possible to more accurately diagnose the site of lesion EP 2 052 673 discloses a method for operating an endoscope in which the target is illuminated by a first light component and a second light component one after another by means of a rotating filter wheel.

However, in special light observation, an image which is captured at a time when narrow-band light is applied to the body tissue is observed. Therefore, even if the illuminance of narrow-band light is appropriately adjusted during short-distance observation, sufficient illuminance to observe the blood vessel in the superficial layer is not obtained during long-distance observation in which the angle of view is wide. Therefore, whenever the observation conditions, such as an observation object and a position, are changed, a gain of an imaging device and/or a display device is adjusted and observation is performed at an appropriate brightness level. The endoscope disclosed in JP 2002-34893 A switches light emitted from the white light source in a time division manner using color filters so as to emit light (R light, G light, and B light) in different wavelength bands in a frame sequential manner and captures an image. Therefore, in order to obtain a full color observation image in real time, it is necessary to combine a plurality of frames (an R frame, a G frame, and a B frame) of captured images, and it is difficult to increase the frame rate of the observation images.

In accordance with the preamble of claim 1, EP 2 052 673 A1 discloses an endoscope having a light source section comprising a white light xenon lamp and a rotary filter such that during operation either white light or narrow-band light passes the rotary filter.

US 2007/203413 A1 discloses an endoscope using a near-infrared camera and a video camera capturing images which are displayed side by side. The images can also be superimposed. Images may be obtained by the two cameras with different frame rates.

SUMMARY OF THE INVENTION

An endoscope in accordance with the invention is defined in claim 1.

With the endoscope of the invention, it is possible to constantly obtain an observation image which is generated by the narrow-band light and which has an appropriate brightness level even though observation conditions, such as an observation object and an observation position, are changed during special light observation by the endoscope, and it is also possible to clearly observe body information obtained by the narrow-band light.

BRIEF DESCRIPTION OF THE DRAWINGS

Fig. 1 is a conceptual block diagram illustrating the structure of an endoscope according to an embodiment of the invention.
Fig. 2 is a diagram illustrating an example of the appearance of the endoscope shown in Fig. 1.
Fig. 3 is a graph illustrating emission spectrums of a purple laser beam emitted from a purple laser beam source, a blue laser beam emitted from a blue laser beam source, and light into which the blue laser beam is wavelength-converted by a phosphor.
Fig. 4 is a diagram illustrating image signals before and after a correction process is performed by a matrix calculating section.
Fig. 5 is a diagram schematically illustrating a blood vessel in a superficial layer of the mucous membrane in the body tissue.
Fig. 6 is a diagram illustrating an example of the schematic display of an observation image obtained by the endoscope, and shows a white light observation image and a narrow-band light observation image.
Fig. 7 is a flowchart illustrating a process of controlling a brightness level of the image signal when light is emitted from a white light source and a light source for special light to capture an image.
Fig. 8A is a diagram illustrating an example of captured image data.
Fig. 8B is a diagram illustrating an example of dividing the image into image areas.
Fig. 8C is a diagram illustrating a weighting process according to positions in a screen.
Fig. 8D is a diagram illustrating corrected image data subjected to the weighting process.

Fig. 9A is a diagram illustrating a characteristic image area extracted from image data.

Fig. 9B is a diagram illustrating emphasized image data.

Fig. 9C is a diagram illustrating a captured image after a brightness level is adjusted.

Fig. 9D is a diagram illustrating an image that is adjusted to a target brightness value.

Fig. 10 is a diagram illustrating that captured images are changed so as to be close to target brightness values.

Fig. 11 is a flowchart illustrating a substitute procedure for steps S6 and S7 of Fig. 7.

Fig. 12 is a diagram schematically illustrating another endoscope in which a white light source is changed.

Fig. 13 is a configuration diagram schematically showing another endoscope using a white light source and a laser beam source.

Fig. 14 is a diagram illustrating the structure of a CMOS image sensor.

## DETAILED DESCRIPTION OF EMBODIMENTS OF THE INVENTION

Hereinafter, exemplary embodiments of the invention will be described in detail with reference to the accompanying drawings.

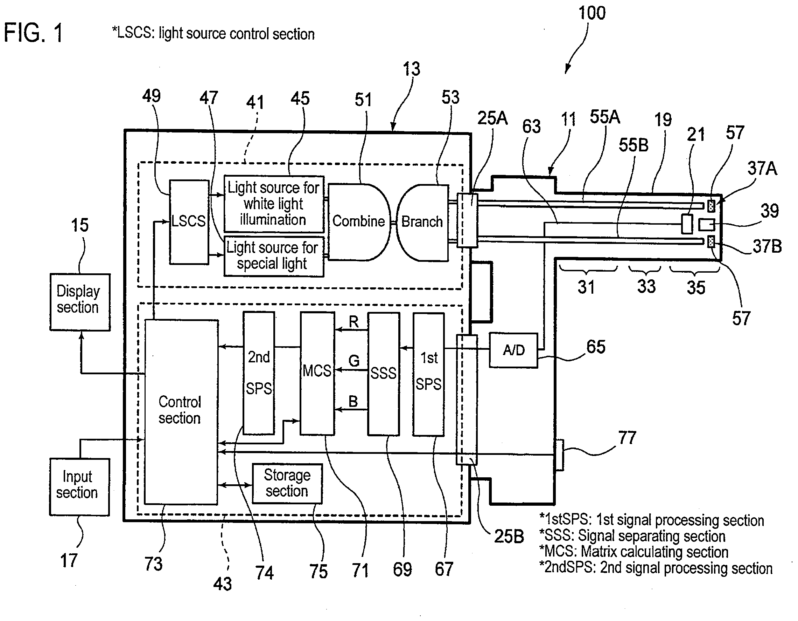

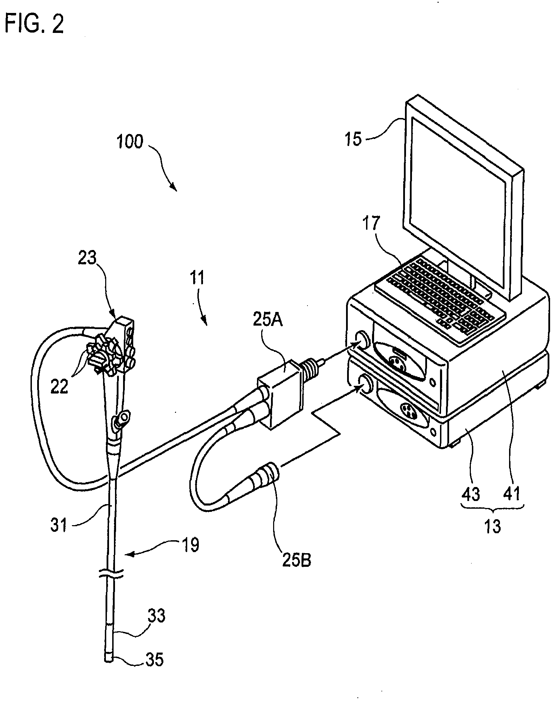

Fig. 1 is a conceptual block diagram illustrating the structure of an endoscope according to an embodiment of the invention. Fig. 2 is a diagram illustrating the appearance of an example of the endoscope shown in Fig. 1.

As shown in Figs. 1 and 2, an endoscope 100 includes an endoscope 11, a control device 13 that is connected to the endoscope 11, a display section 15 that is connected to the control device 13 for display of image information, and an input section 17 that receives an input operation. The endoscope 11 is an electronic endoscope including an illumination optical system that emits illumination light from the leading end of an endoscope insertion portion 19 which is inserted into a body to be examined and an imaging optical system including an imaging device 21 (see Fig. 1) that has detection pixels of plural colors and captures an image of a region to be observed.

The endoscope 11 includes the endoscope insertion portion 19, an operating section 23 (see Fig. 2) that performs an operation for bending the leading end of the endoscope insertion portion 19 and/or an operation for observation, and connector sections 25A and 25B that removably connect the endoscope 11 to the control device 13. Although not shown in the drawings, various kinds of channels, such as a forceps channel through which a treatment tool for tissue collection is inserted, a channel for air supply, and a channel for water supply, may be provided in the operating section 23 and the endoscope insertion portion 19.

The endoscope insertion portion 19 includes a flexible portion 31, a bending portion 33, and a leading end portion (hereinafter, also referred to as an "endoscope leading end portion") 35. As shown in Fig. 1, emission ports 37A and 37B through which light is emitted to a region to be observed and an imaging device 21, such as a CCD (Charge Coupled Device) type image sensor or a CMOS (Complementary Metal-Oxide Semiconductor) type image sensor that acquires image information of the region to be observed, are provided in the endoscope leading end portion 35. Also, an objective lens unit 39 is provided on a light receiving surface of the imaging device 21. Where the CCD type image sensor is adopted, since it uses the global shutter, an image having less image skew and low noise can be achieved.

The bending portion 33 is provided between the flexible portion 31 and the leading end portion 35 and can be bent by rotating an angle knob 22 which is provided in the operating section 23 shown in Fig. 2. The bending portion 33 can be bent in an arbitrary direction and at an arbitrary angle according to a part of the object to be examined by the endoscope 11 so that the observation direction of the imaging device 21 and the emission ports 37A and 37B of the endoscope leading end portion 35 can face a desired observation part. Although not shown in the drawings, a cover glass or a lens is provided over the emission ports 37A and 37B of the endoscope insertion portion 19.

The control device 13 includes a light source device 41 that emits illumination light to the emission ports 37A and 37B of the endoscope leading end portion 35, and a processor 43 that performs image processing on captured image signals from the imaging device 21. The control device 13 is connected to the endoscope 11 through the connector sections 25A and 25B. The display section 15 and the input section 17 are connected to the processor 43. The processor 43 performs image processing on the captured image signals transmitted from the endoscope 11 to generate a display image and supplies the display image to the display section 15, based on commands from the operating section 23 and/or the input section 17 of the endoscope 11.

The light source device 41 includes, as light sources, a blue laser beam source (a light source for white light illumination) 45 having a central wavelength of 445 nm and a purple laser beam source (light source for special light) 47 having a central wavelength of 405 nm. A light source control section 49 individually controls emission of light from semiconductor light emitting elements of the light sources 45 and 47 to freely change a ratio of an amount of light emitted from the blue laser beam source 45 to an amount of light emitted from the purple laser beam source 47.

Broad-area InGaN laser diodes may be used as the blue laser beam source 45 and the purple laser beam source 47. Also, InGaNAs laser diodes or GaNAs laser diodes may be used. A light emitter, such as a light emitting diode, may be used as any of the light sources.

The laser beams emitted from the light sources 45 and 47 are input to an optical fiber by a condensing lens (not shown) and are transmitted to the connector section 25A through a combiner 51, which is a multiplexer, and a coupler 53, which is a optical branching filter. However, the invention is not limited thereto. The laser beams emitted from the light sources 45 and 47 may be directly transmitted to the connector section 25A without using the combiner 51 and the coupler 53.

The blue laser beam having the central wavelength of 445 nm and the purple laser beam having the central wavelength of 405 nm are combined and transmitted to the connector section 25A, and the combined laser beams are transmitted to the endoscope leading end portion 35 of the endoscope 11 through optical fibers 55A and 55B. Then ,the blue laser beam excites phosphors 57, which are wavelength conversion members and which are provided at the light emission ends of the optical fibers 55A and 55B of the endoscope leading end portion 35, and fluorescence is emitted. Also, some of the blue laser beams pass through the phosphor 57. The purple laser beam passes through the phosphor 57 without exciting the phosphor 57 and becomes illumination light having a narrow band wavelength.

The optical fibers 55A and 55B are multi-mode fibers. For example, a thin fiber cable having a core diameter of 105 $\mu$m, a clad diameter of 125 $\mu$m, and a diameter $\phi$ of 0.3 mm to 0.5 mm including a protective layer, which is an outer cover, may be used as the optical fiber.

The phosphor 57 includes plural kinds of phosphors (for example, a YAG phosphor or a phosphor such as BAM ($BaMgAl_{10}O_{17}$)) that absorb some of the blue laser beams to be excited and emit green to yellow light. In this way, green to yellow excitation light generated by excitation with the blue laser beam and the blue laser beam which passes through the phosphor 57 without being absorbed are combined to produce white (pseudo-white) illumination light. As in this embodiment, when the semiconductor light emitting element is used as an excitation light source, it is possible to obtain high-intensity white light at high emission efficiency. Therefore, it is possible to easily adjust the intensity of white light and reduce a variation in color temperature and chromaticity of the white light.

The phosphor 57 can prevent the superposition of noise, which is an obstruction to image capture, or the occurrence of flicker during the display of a moving picture which are caused by speckles generated by the coherence of a laser beam. Also, it is preferable that the phosphor 57 be configured in the following manner. That is, considering a difference between a refractive index of the fluorescent material constituting the phosphor and a refractive index of a fixing/solidifying resin, which is a filler, the fluorescent material and the filler have particle diameters that absorb a small amount of light in the infrared region and largely scatter light in the infrared region. In this way, it is possible to improve the scattering effect without reducing the intensity of red light or light in the infrared region and reduce an optical loss.

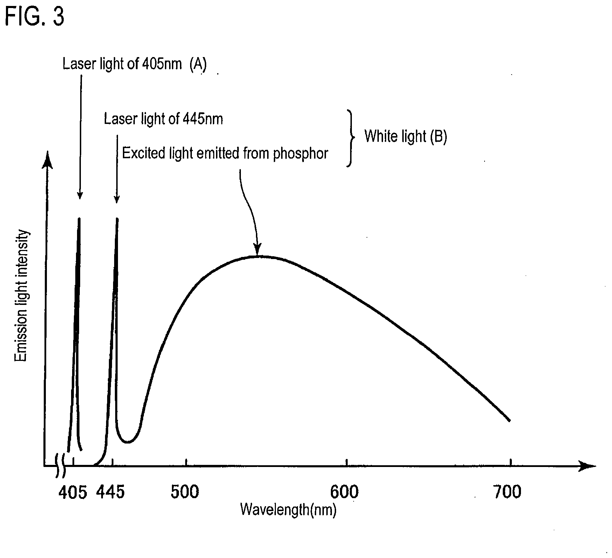

Fig. 3 is a graph illustrating the emission spectrums of the purple laser beam emitted from the purple laser beam source 47, the blue laser beam emitted from the blue laser beam source 45, and light whose wavelength is converted from the blue laser beam by the phosphor 57. The purple laser beam is represented by an emission line (profile A) having the central wavelength of 405 nm. The blue laser beam is represented by an emission line having the central wavelength of 445 nm, and the light excited from the phosphor 57 by the blue laser beam has a spectral intensity distribution in which the emission intensity thereof increases in a wavelength band of about 450 nm to 700 nm. The white light is formed of a profile B of the excitation light and the blue laser beam.

In the specification, strictly, the white light is not limited to light including all wavelength components of visible light. For example, the white light may include light components in a specific wavelength band, such as R (red), G (green), and B (blue), which are the reference colors. Also, the white light may include, for example, light including wavelength components in the range from green to red or light including wavelength components in the range from blue to green in a broad sense.

In the endoscope 100, the light source control section 49 can relatively increase or decrease the emission intensities of the profile A and the profile B to generate illumination light having an arbitrary brightness balance.

This embodiment will be described with reference to Fig. 1 again. As described above, illumination light including (i) the white light formed of the blue laser beam and the excitation light which is produced by the phosphor 57 and (ii) narrow-band light of the purple laser beam is emitted from the leading end portion 35 of the endoscope 11 to a region of the object to be observed. Then, the objective lens unit 39 forms an image of the region to be observed to which the illumination light is emitted on the light receiving surface of the imaging device 21, and the imaging device 21 captures the image.

After an image is captured, the captured image signal output from the imaging device 21 is transmitted to an A/D converter 65 through a scope cable 63 and is then converted into a digital signal. Then, the digital signal is input to a first signal processing section 67 of the processor 43 through the connector section 25B. The first signal processing section 67 performs, for example, white balance correction and contour enhancement on the image signal, which is output from the imaging device 21 and then converted into the digital signal by the A/D converter 65. The image signal processed by the first signal processing section 67 is transmitted to a signal separating section 69 and is then divided into R, G, and B image signals. Then, the separated image signals are transmitted to a matrix calculating section 71.

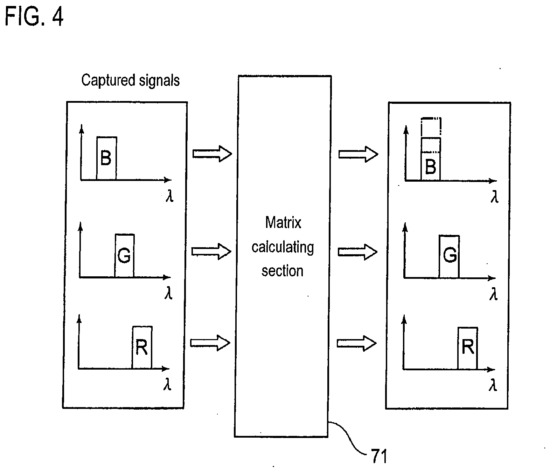

Fig. 4 shows the image signals before and after a correction process is performed by the matrix calculating section 71.

For each captured image signal formed of the R, G, and B signals supplied from the signal separating section 69, the matrix calculating section 71 individually increases or decreases the brightness level of each of the R, G, and B signals in accordance with commands from a control section 73. The matrix calculating section 71 stores a correction matrix which is used to individually correct the brightness value of each color of the captured image signal. The matrix calculating section 71 performs the matrix operation on the brightness values of each captured image using the correction matrix, to thereby be able to finely correct the brightness level of the captured image for each color. The correction matrix (Cr, Cg, Cb) can be represented by the following expression (1):

$$\begin{pmatrix} r \\ g \\ b \end{pmatrix} = \begin{pmatrix} Cr & Cg & Cb \end{pmatrix} \begin{pmatrix} R \\ G \\ B \end{pmatrix} \qquad (1)$$

where R, G, and B denote the brightness levels of an input signal, and r, g, and b denote brightness levels of a corrected captured image.

Te captured image signals whose brightness levels are controlled by the matrix calculating section 71 are transmitted to the second signal processing section 74. The second signal processing section 74 converts the image signal transmitted from the matrix calculating section 71 into a video signal, such as a video composite signal or a Y/C separated signal, performs D/A conversion on the video signal to generate image data for display, and outputs the converted analog signal to the display section 15 at any time.

As described above, the first signal processing section 67, the signal separating section 69, the matrix calculating section 71, and the second signal processing section 74 convert an input digital captured image signal into image data and output desired image information for output to the control section 73.

The image information for output input to the control section 73 is displayed as an endoscope observation image on the display section 15 and is stored in a storage section 75, which is a memory or a storage device, if necessary. A mode change button 77, which will be described in detail later, is provided in the endoscope 11. A switching signal from the mode change button 77 is input to the control section 73.

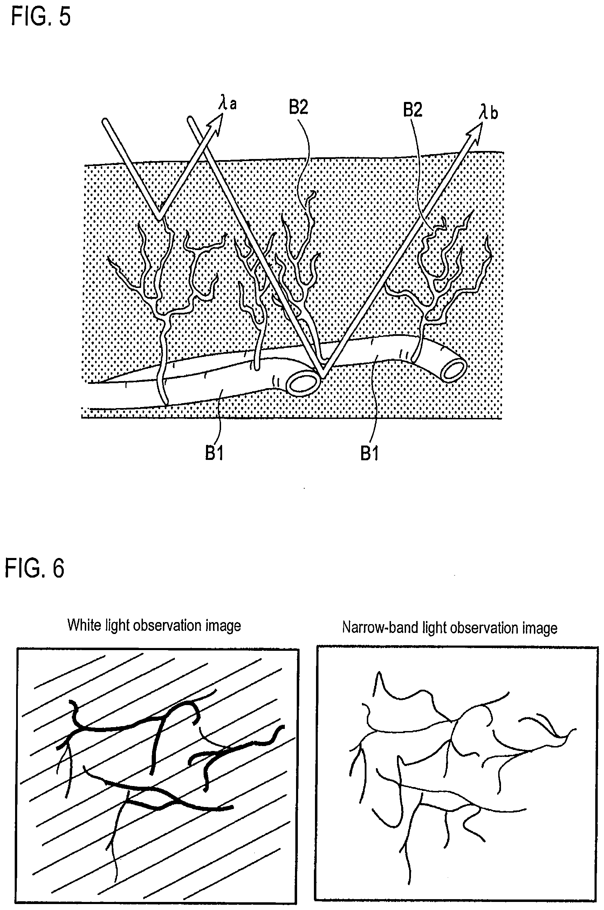

Fig. 5 is a diagram schematically illustrating the blood vessel in a superficial layer of the mucous membrane of the body tissue. In the superficial layer of the mucous membrane of the body tissue, a capillary vessel B2, such as a resinous blood vessel network, is formed between a blood vessel B1 in a deep layer of the mucous membrane and the superficial layer of the mucous membrane. It has been reported that the lesion of the body tissue appears in a microscopic structure such as the capillary vessel B2. In endoscopy, there is an attempt to emphasize and observe an image of the capillary vessel in the superficial layer of the mucous membrane using visible short-wavelength light of the narrow wavelength band, such as blue to purple light, thereby detecting a microlesion early or diagnosing the range of lesion.

When illumination light is incident on the body tissue, the incident light diffusely propagates in the body tissue. The absorption and scattering characteristics of the body tissue have wavelength dependency, and the scattering characteristics tend to increase as the wavelength becomes short. That is, the invasion depth of light varies depending on the wavelength of the illumination light. Blood vessel information is obtained from the capillary vessel in the superficial layer of the mucous membrane when illumination light is in the wavelength band $\lambda_a$ of about 400 nm, and blood vessel infonnation including the blood vessel in the deep layer of the mucous membrane is obtained when illumination light is in the wavelength band $\lambda_b$ of about 500 nm. Therefore, a light source having a central wavelength of 360 nm to 800 nm, preferably, 365 nm to 515 nm is used to observe the blood vessel of the body tissue. In particular, a light source having a central wavelength of 360 nm to 470 nm, preferably, 360 nm to 450 nm is used to observe the blood vessel in the superficial layer.

Fig. 6 is a diagram schematically illustrating a display example of an observation image by the endoscope. In a white light observation image which is obtained when white light is used as illumination light, it is possible to obtain the image of the blood vessel in the deep layer of the mucous membrane, and it is easy to improve the brightness of the entire image. Meanwhile, in a narrow-band observation image which is obtained when narrow-band light including a lot of short-wavelength visible light components is used as illumination light, the capillary vessels in the superficial layer of the mucous membrane are clearly observed.

When the observation image obtained by the white light and the observation image obtained by the narrow-band light are combined, it is possible to ensure sufficient brightness of the entire image, and an observation image is obtained in which the microscopic vessel in the superficial layer of the mucous membrane of the body tissue is emphasized, which makes it easy to diagnose a diseased part. In the endoscope 100 having the above-mentioned structure, the narrow-band light having the profile A and the white light having the profile B shown in Fig. 3 are applied from the endoscope leading end portion 35 to a region to be observed, and the imaging device 21 captures reflected light of the illumination light to acquire image signals of one frame in which both of the narrow-band light and the white light are included. Then, the intensities of the R signal, G signal, and B signal of the image signal are individually controlled to generate an observation image. The obtained observation image becomes a captured image in the illuminated state in which a ratio of the amount of the narrow-band light having the profile A to the amount of the white light having the profile B is changed in a pseudo manner.

That is, the observation image obtained by the narrow-band light having the central wavelength of 405 nm includes its main imaging information in the B signal of the imaging device 21. The observation image, which is obtained by the blue laser beam having the central wavelength of 445 nm and the white light generated by the light emission of the phosphor includes imaging information in the respective captured images of the R signal, G signal, and B signal of the imaging device 21. Then, it is possible to emphasize or blur only the imaging information obtained by the narrow-band light in the observation image by changing the brightness level of the captured image of the B signal in the captured image signal relatively to the brightness levels of the captured images of the R and G signals.

As such, if the brightness values of the captured images of the R signal, G signal and B signal output from the imaging device 21 are varied continuously and independently when light components of the both illumination light are included in the captured image of one frame, an observation image can be generated in which both of the observation image of the narrow-band light and the observation image of the white light are combined appropriately without the observation image of the white light hindering the observation image of the narrow-band light. Thereby, the blood vessel in the superficial layer is emphasized by the narrow-band light while the entire periphery of an observation portion is brightly illuminated by white light. It is possible to capture an observation image that facilitates the observation of the structure of the microscopic vessel.

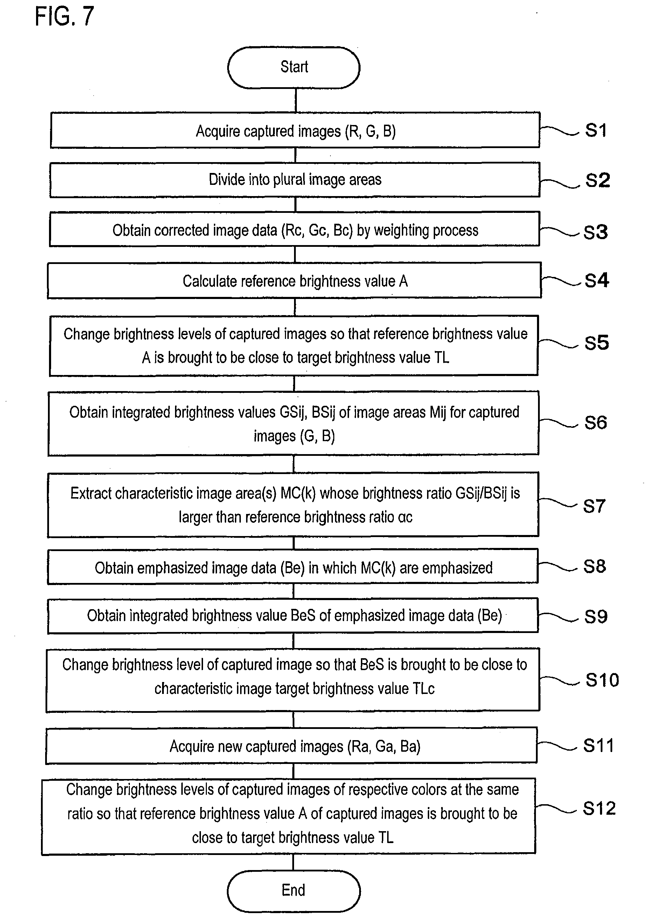

Next, with reference to a flowchart of Fig. 7, described will be given on a process of controlling the brightness level of an image signal when imaging is performed under illumination by (i) the blue laser beam source 45, which is a light source for white light illumination, and (ii) the purple laser beam source 47, which is a light source for special light, in order to appropriately maintain the average brightness of the entire captured image and a mixture balance between an image formed by the narrow-band light and an image formed by the white light when imaging in the case where imaging is performed under illumination light including not only the narrow-band light but also the white light as described above.

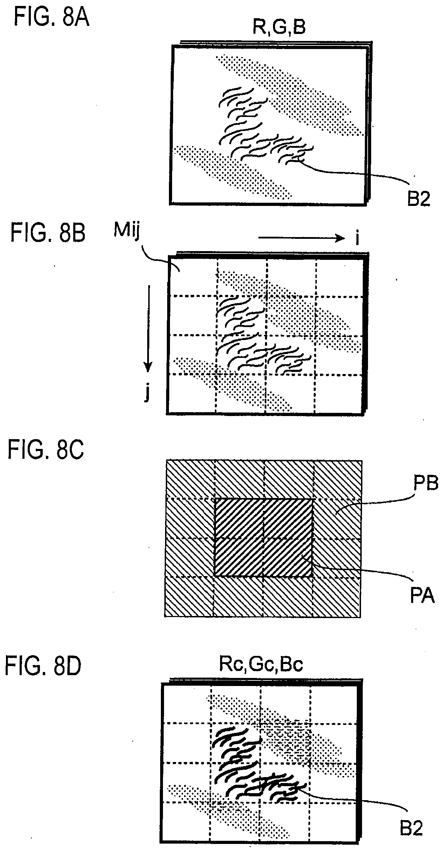

First, both the blue laser beam source 45 and the purple laser beam source 47 shown in Fig. 1 are turned on to emit narrow-band light and white light to a region to be observed. In this state, the imaging device 21 captures the image of the region to be observed. The captured image signal which is obtained in this manner is input to the first image processing section 67 to acquire respective captured images (R, G, B) having a predetermined tone expression range, as shown in Fig. 8A (S1). The respective captured images (R, G, B) are image information which are obtained at the same capturing timing.

When the imaging device 21 is a primary-color-based imaging device, detection levels of R, G, and B which are detection colors are treated as the brightness values of the reference colors (R, G, B). However, when the imaging device 21 is a complementary-color-based imaging device, detection levels of three colors of C (cyan), M (magenta), and Y (yellow) or four colors of C, M, Y, and G are calculated and converted into the brightness values of the reference colors of R, G, and B.

The conversion from CMY or CMYG into RB is executed by the first signal processing section 67 based on a predetermined operation expression or a table. That is, the captured images (C, M, Y) or (C, M, Y, G) subjected to the A/D conversion are converted into signals of the captured images (R, G, B) of the reference colors. In blue which has the shortest wavelength among the reference colors of the captured images (R, G, B), information of a blood vessel B2 in the superficial layer (see Fig. 5) acquired by the narrow-band light having 405 nm at the center wavelength is displayed at a center of a screen.

The signal separating section 69 outputs the captured images (R, G, B) of the reference colors to the matrix calculating section 71 color by color, and the matrix calculating section 71 divides the captured images (R, G, B) into an arbitrary number of image areas $M_{ij}$, as shown in Fig. 8B (S2). In the example shown in Fig. 8B, the captured images (R, G, B) are commonly divided into 16 image areas in total, that is, $4 \times 4$ image areas ($M_{ij}$: i = 0 to 3, j = 0 to 3). It should be noted that the number of divided image areas may be any value.

Then, the control section 73 performs a weighting process for each of the image areas $M_{ij}$ of the captured images (R, G, and B) to obtain corrected image data (Rc, Gc, Bc) (S3). As shown in Fig. 8C, the weighting process is a process to emphasize an image area PA, which is disposed at the center of the screen, of the captured images (R, G, B), as compared to an image area PB which is disposed in the periphery of the screen. The weighting process emphasizes an object to be particularly observed that is displayed at the center of the screen. The brightness value of each captured image is corrected by a matrix operation using a correction matrix. As shown in Fig. 8D, the capillary vessel B2 in the superficial layer of the tissue, which is a main observation object, is emphasized in the corrected image data (Rc, Gc, Bc) obtained by performing the weighting process for the captured images (R, G, B).

Then, a reference brightness value A indicating a brightness with respect to the entire image of the corrected image data (Rc, Gc, Bc) is calculated (S4). For example, as in the following expression (2), the reference brightness value A is an index obtained by averaging the brightness values of the pixels of the corrected image data (Rc, Gc, Bc) with respect to all pixels (N pixels).

$$A = \frac{\sum_N Rc + \sum_N Gc + \sum_N Bc}{3N} \qquad (2)$$

The control section 73 changes the brightness levels of the captured images (R, G, B) so that the reference brightness value A obtained from the corrected image data (Rc, Gc, Be) is brought close to a predetermiend target brightness value TL1 (S5). That is, the control section 73 compares the obtained reference brightness value A with the target brightness value TL, which is stored in the storage section 75 in advance, and sets the correction matrix stored in the matrix calculating section 71 again so that the reference brightness value A is brought close to the target brightness value TL. That is, the control section 73 generates control signals for increasing or decreasing the brightness levels of the captured images (R, G, B) under the respective illumination light, and outputs the control signals to the matrix calculating section 71.

The matrix calcualting section 71 adjusts the stored correction matrix based on the control signals sent from the control section 73, and executes a correction process for the captured images (R, G, B) using the adjusted correction matrix, to thereby increase or decrease the brightness levels of the captured images (R, G, B). Thereby, if the reference brightness value A is smaller than the target brightness value TL, the brightness values of the captured images (R, G, B) are increased, and if the reference brightness value A exceeds the target brightness value TL, the brightness values of the captured images (R, G, B) are decreased.

The control of the brightness levels of the captured images is performed by changing the correction matrix (Cr, Cg, Cb) for the respective captured images (R, G, B) in the matrix calculating section 71 and by executing the matrix operation using the changed correction matrix. The control is synonymous with setting amplification gains for the brightness values of the respectove captured images (R, G, B) again. In the subsequent capturing, captured image signals are corrected using the reset gainst, that is, the reset correction matrix, and the corrected captured images (R, G, B) are output from the matrix calculating section 71. It is noted that the set correction matrix is further reset again in the process described below.

Then, of the captured images (R, G, B) acquired at S1, the control section 73 calculates integrated brightness values $GS_{ij}$ and $BS_{ij}$ of the respective pixels in the divided image areas $M_{ij}$ of the captured images (G, B) (S6). That is, the control section 73 calculates the integrated brightness values $GS_{ij}$ and $BS_{ij}$ for the 16 image areas $M_{ij}$ of the captured images (G, B).

Then, the control section 73 calculates a brightness ratio $\alpha$, which is a ratio of the integrated brightness values $GS_{ij}$ and $BS_{ij}$ in the image areas $M_{ij}$ havivng the same image positional relationship using the following expression (3). Then, the control section 73 extracts an image area whose brightness ratio $\alpha$ is larger than a reference brightness ratio $\alpha c$ as a characteristic image area MC(k) (S7).

$$\alpha = \frac{GS_{i,j}}{BS_{i,j}} \qquad (3)$$

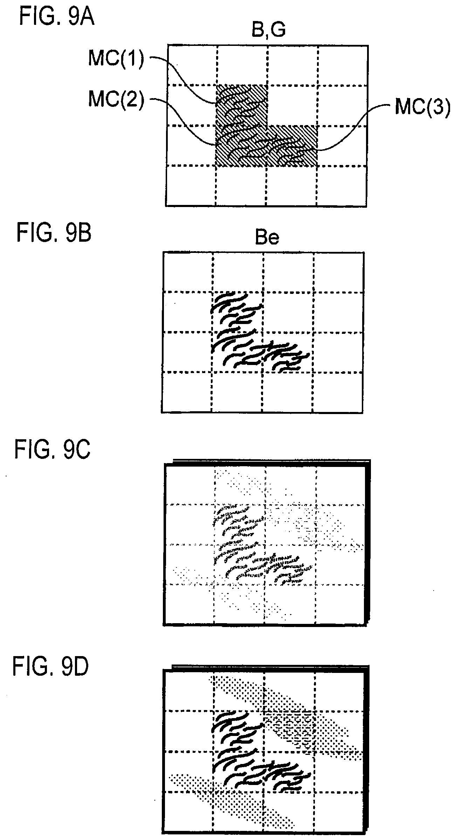

Fig. 9A shows the characteristic image area MC(k) extracted from the captured images (B and G) (for example, three areas: k = 1, 2, 3).

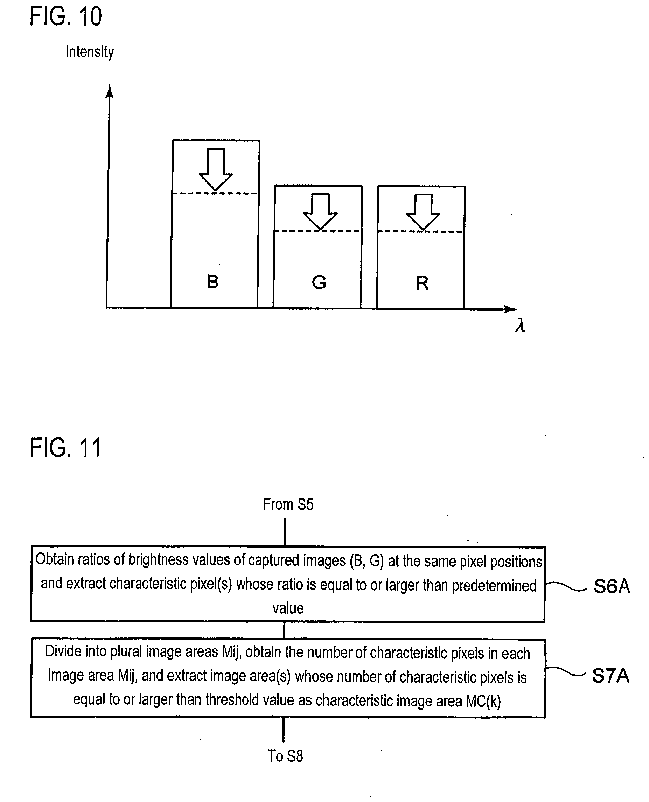

Then, in particular, for the captured image (B) which includes a large amount of information about the capillary vessel B2 in the superficial layer of the tissue emphasized by narrow-band light having 405 nm at the center wavelength, each pixel of the extracted characteristic image area MC(k) is emphasized by the weighting process to calculate emphasized blue image data (Be), as shown in Fig. 9B (S8). The emphasized image data (Be) is an image in which only the characteristic image area MC(k) is emphasized. In the example shown in Fig. 10B, the image of a portion in which the capillary vessel B2 appears is emphasized so as to have higher brightness than the other image areas.

Then, a integrated brightness value BeS of the emphasized image data (Be) in the entire screen is calculated by the following expression (4) (S9).

$$BeS = \sum_N Be \qquad (4)$$

The control section 73 generates control signals for increasing or decreasing the brightness level of the caotyred image (B) including the imaging information obtained by the narrow-band light having 405 nm at the center wavelength so that the obtained integrated brightness value BeS is brought close to a predetermined characteristic image target brightness value TLc, and outputs the control signals to the matrix calculating section 71. Then, when the integrated brightness value BeS is smaller than the characteristic image target brightness value TLc, the matrix caluclating section 71 increases the brightness level of the captured image (B), and when the integrated brightness value BeS exceeds the characteristic image target brightness value TLc, the matrix calculating section decreases the brightness level of the captured image (B). The increase/decrease control of the brightness level is performed by resetting the correcction matrix (gain values) for the respective captured images (S10).

After the correction matrix is adjusted so that the captured image (B) has a desired brightness level, the imaging device 21 acquires captured image singlas and generates captured images (Ra, Ga, Ba) again (S11). Then, the control section 73 obtains the reference brightness value A of the captured images (Ra, Ga, Ba) by the weighting process for each image area and by.the expression (2). An example of the captured images (Ra, Ga, Ba) at this time is shown in Fig. 9C. Fig. 9C shows that case where the brightness level of the captured image (Ba) is increased, and the brightness levels of the captured images exceed the maximum tone expression range.

In the case where the reference brightness value A exceeds the target brightness value TL, for example, in the case where the brightness levels of the captured images exceed the maximum tone expression range as a result of the adjustment control of the brightness levels as described above, it is necessary to make a correction. In this case, the brightness levels of the respective captured images (R, G, B) are decreased at the same ratio for the respective colors.

Fig. 10 is a diagram illustrating that captured images are changed so as to be close to the target brightness value TL. As shown in Fig. 10, the brightness levels of the captured images of R, G, B are adjusted at the same ratio for the respective colors so as to be the target brightness value TL (S12). This adjustment is also made by resetting the correction matrix included in the matrix calculating section 71 and executing the matrix operation again using the reset correction matrix.

Thereby, as shown in Fig. 9, an observation image is obtained in which both of the image infomraiton by the white color and the image infomration by the narrow-band light which is controlled to be a desired brightness level have appropriate brightness levels over the entire screen.

Accordignly, since the narrow-band light and the white light are emitted at the same time to continuously capture images, the performance of tracking the movement of an observation target is improved. Therefore, it is possible to emphasize and display, for example, an image of a blood vessel in a superficial layer obtained by the narrow-band light while ensuring a brightness of an observation image obtained by the white light. That is, both a white light source for general observation and a light source for special light observation emit light to capture an image, and it is possible to obtain an observation image having a brightness value that is not saturated in the entire observation image, that is, does not exceed the maximum tone expression range while an object (for example, the blood vessel in the superficial layer or the duct of the gland) that is to be observed with the narrow-band light has an optimal brightness level. Thereby, it is possible to constantly display the observation object at an appropriate brightness level. In particular, it is possible to clearly display an observation part emphasized by the narrow-band light without being hindered (concealed) by the white light. Therefore, an endoscope observation image which contributes to early detection of an involved site can be achieved without an operator's adjsutment operation.

Then, the observation image makes it possible to simultaneously observe the detailed structure of the superficial layer of the tissue emphasized by narrow-band light in real time while viewing the structure of the entire observation part with white illumination light. Therefore, for example, in the site of cancer lesion in which the density of the microscopic vessel is higher than that in a normal part, it is possible to accurately determine the state of a microscopic vessel or a microscopic structure in the superficial layer while contrasting the site of lesion with a peripheral portion thereof.

In this embodiment, the blue laser beam source 45 and the purple laser beam source 47 are turned on at the same time to capture images. Alternatively, the blue laser beam source 45 and the purple laser beam source 47 may be alternately turned on during a light receiving period in one frame of the imaging device. In this case, it is possible to reduce power consumption or the generation of heat.

Also, it is possible to press the mode change button 77 (see Fig. 1) to turn on/off the function of controlling the brightnss level of the captured image signal. When the function is turned on, the operation mode is changed to the special light observation mode, and it is possible to observe an object with both the white light and the narrow-band light. When the function is turned off, the operation mode is changed to the normal observation mode, and it is possible to change the brightness level of the image signal obtained by the purple laser beam source 47 and the brightness level of the image signal obtained by the blue laser beam source 45 at the same ratio. As such, it is possible to improve the use convenience of the endoscope by selectively switching the operation mode between the special light observation mode and the normal observation mode.

Furthermore, an object to be observed with the narrow-band light may not only be a capillary vessel in a superficial layer of a tissue or fine mucosal pattern, but also be autofluorescence or drug fluorescence. The embodiment can appropriately change an intensity of light returning from a region to be observed, so as to be suitable for diagnosis.

The method for extracting the characteristic image area(s) MC(k) may be performed in the following manner.

That is, the above-described characteristic image areas MC(k) are extracted by comparing the brightness values of B and G in units of divided image areas in the captured images (B, G). However, when the brightness values of B and G are compared in units of pixels in the captured images, an object to be emphasized by the narrow-band light can be extracted more precisely.

Specifically, S6 and S7 of Fig. 7 are replaced with S6A and S7A shown in a flowchart of Fig. 11. With respect to the captured image (B) which contains much information about reflection light of the narrow-band light having 405 nm at the center wavelength and the captured image (G), a ratio of the brightness values of the captured images (B, G) at each pixel position is obtained, and a characteriestic pixel(s) whose ratio is euqal to or larger than a predetermined ratio are extracted (S6A).

As shown in Fig. 8 which has already been described, the captured image (B) and the captured image (G) are commonly divided into plural image areas $M_{ij}$, and the number of extracted caracteristic pixels is obtained for each image area $M_{ij}$. Then, an image area(s) whose number of characteristic pixels is equal to or larger than a predetermined threshold value are extrated as the characteristic image areas MC(k) (S7A).

When the brightness values of B, G at the same pixel positions are compared in units of pixels as described above, body information obtained by the narrow-band light can be extracted more surely.

Next, another configuration example of an endoscope will be described.

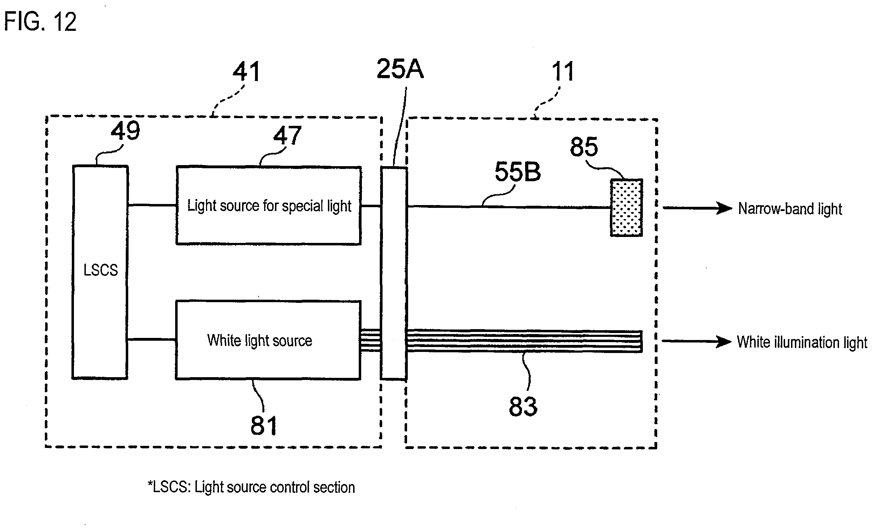

Fig. 12 is a diagram schematically illustrating the structure of another endoscope in which the white light source is modified. In the example shown in Fig. 12, a light source that emits light in a broad wavelength band, such as a halogen lamp, a xenon lamp, or a white light emitting diode, is used as a white light source 81. The white light source 81 emits white light from a leading end portion of the endoscope 11 through a light guide 83, which is an optical fiber bundle. Light emitted from the light source 47 for special light is transmitted to the leading end portion of the endoscope 11 through the connector section 25A by the optical fiber 55B, similar to the above-described embodiment. Then, the light is supplied as narrow-band light by a light polarization/diflusion member 85 that is provided at a light emission end of the optical fiber 55B. The light polarization/diffusion member 85 may be replaced with a light emission window that is provided at the leading end portion of the endoscope 11.

With the above-mentioned structure, it is possible to introduce white illumination light having a broad spectral characteristic and a high color rendering property with a simple structure. Furthermore, the generation of heat in the leading end portion of the endoscope can be suppressed. In addition, it is possible to emit white light and narrow-band light such that they are completely separated from each other. Therefore, the narrow-band light can be applied to a region to be observed without passing through a phosphor. Accordingly, unnnecessary light emission from the phosphor can be eliminated, and light amount control can be easily made.

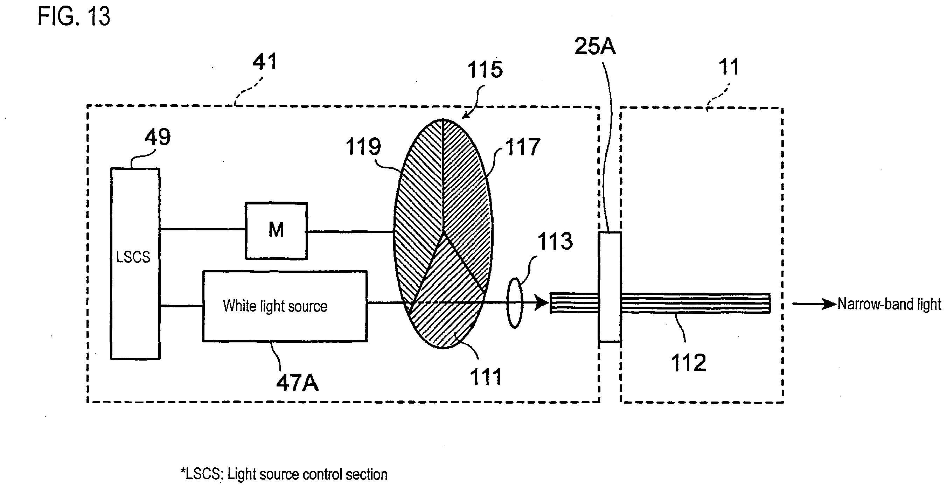

Fig. 13 is a configuration diagrapm schematically showing another endoscope in which a light source for special light is modified. In the figure, an optical system for the whilte illumination light is not shown in detail, and the one shown in Fig. 1 or Fig. 12 may be used. In the light source for special light of this example, in place of the purple laser light source 47, which emits the narrow-band light, a white light source 47A, such as a halogen lamp, a xenon lamp or a white light emitting diode, that emits light having a broad wavelength band and an optical filter 111 are used to generaete narrow-band light. Then, transmission light from the optical filter 111 is introduced into an optical incident end of a light guide by a light converging member, and is guided to the leading end portion of the endoscope 111 by the light guide 112.

The optical filter 111 is a narrow-band pass filter that only allows predetermined narrow-band wavelength components of the incident whilte light to pass therethrough. The optical filter 111 is formed in a part of a rotation filter plate 115. The rotation filter plate 115 can change an optical filter arranged on an optical path of the white light by rotary driving performed by a mortor M. That is, plural types of optical filters 111, 117 and 119 (the number of optical filters is not limtied to three as in this example, but may be any number) are arranged on the optical paht in a swithcing manner, to thereby emit different types of narrow-band light.

With the abvoe configuraiton, arbitrary narrow-band light can be easily genrated from the white light soruce.

In the above-described embodiment, the brightness level of the image signal output from the imaging device is controlled to increase or decrease. The brightness level of the image signal may be controlled to increase or decrease inside the imaging device.

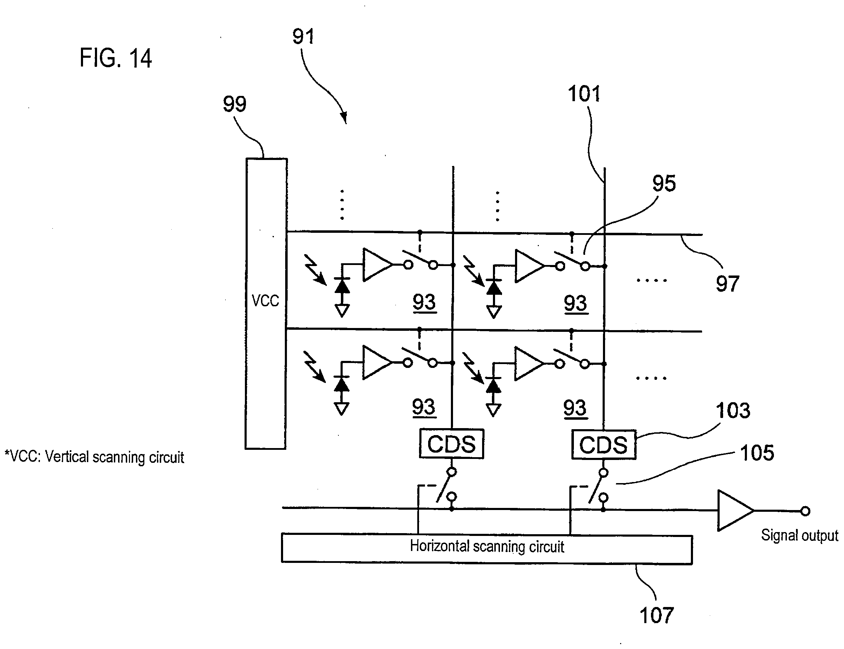

Fig. 14 is a configuration diagram of a CMOS image sensor. A CMOS image sensor 91 is used as the imaging device of the above-mentioned endoscope. In this case, an amplifier 93 of each pixel in the CMOS image sensor 91 can increase or decrease the brightness level of the image signal. In the CMOS image sensor 91, a switch 95 of the amplifier 93 is connected to a vertical scanning circuit 99 through a row circuit 97. The amplifier 93 is connected to a CDS 103 through a column circuit 101, and the CDS 103 is connected to a horizontal scanning circuit 107 through the switch 105.

With the above configuration, it is not necessary to increase or decrease the brightness level of the image signal in the post-process, and it is possible to reduce the control time. In addition, since each pixel can control the intensity of the signal level, it is possible to perform, for example, a control process of setting the gain of a specific region of the image to a desired value at a high speed and thus perform control at a high response speed. Therefore, it is possible to display a high-quality image captured by the endoscope at a high frame rate.

The invention is not limited to the above-described embodiments. Their modifications that can be made by one skilled in the art based on the specification and the known technique also fall within the scope of the invention and are included in a scope which is seeked to be covered, where this scope is defined by the claims.

**Claims**

1. An endoscope (11) having:

    a first light source section (45) adapted to emit white illumination light;
    a second light source section (47) adapted to emit narrow-band light having a wavelength band narrower than that of the white illumination light,

an imaging section adapted to capture a region to be observed by an imaging device (21) having detection pixels of plural colors; and

a control section (73) adapted to perform operations of

causing the imaging section to output a captured image signal including a return light component of the white illumination light from the region to be observed and a return light component of the narrow-band light from the region to be observed;

generating captured images of plural reference colors based on the captured image signal, wherein

a first captured image of the captured images contains mostly the return light component of the narrow-band light emitted from the second light source; and

changing a brightness level of the first captured image independently from brightness levels of the other captured images,

wherein the control section (73) is adapted to perform control such that both the first light source section (45) and the second light source section (47) are turned on simultaneously, and **characterized in that**

the control section (73) is further adapted for dividing the first captured image and a second captured image which has a different reference color from that of the first captured image into common plural image areas;

integrating brightness values in each image area of the first captured image to obtain an integrated brightness value of each image area of the first captured image;

integrating brightness values in each image area of the second captured image to obtain an integrated brightness value of each image area of the second captured image;

obtaining a ratio of (i) the integrated brightness value of each image area of the first captured image and (ii) the integrated brightness value of the image area, having a same image positional relationship with each image area of the first captured image, of the second captured image;

extracting image areas, of the first and second captured images, whose ratio is equal to or larger than a threshold value, as characteristic image areas; and

selectively changing a brightness level of the characteristic image area of the first captured image.

2. The endoscope according to claim 1, wherein a center wavelength of the narrow-band light emitted from the second light source (47) is in a range of 360 nm to 470 nm.

3. The endoscope according to any one of claims 1 and 2, wherein

the changing of the brightness level includes changing a correction matrix which is used to correct brightness values of respective pixels in the captured image signal by a matrix operation,

the control section is further adapted to

correcting a captured image signal, which is newly obtained from the imaging section, using the changed correction matrix.

4. The endoscope according to claim 3, wherein the control section is further adapted to

if a brightness level of an image obtained by correcting the captured image signal, which is newly obtained from the imaging section, using the changed correction matrix exceeds a predetermined target brightness level, resetting the correction matrix so as to decrease the brightness level of the corrected image.

5. The endoscope according to any one of claims 1 to 4, wherein

light components of detection colors which are detected by the imaging device include light components of a primary color system containing blue, green and red,

the reference color of the first captured image is blue, and

the reference color of the second captured image is green.

6. The endoscope according to any one of claims 1 to 4, wherein

light components of detection colors which are detected by the imaging device include light components of a complementary color system containing magenta, cyan and yellow,

the light components of the respective detection colors are converted into light components of a primary color system of blue, green and red,

the reference color of the first captured image is blue, and

the reference color of the second captured image is green.

7. The endoscope according to any one of claims 1 to 6, wherein the imaging device (21) includes a CCD-type image sensor.

8. The endoscope according to any one of claims 1 to 6, wherein

the imaging device (21) includes a CCD-type image sensor, and

the brightness level of the first captured image is changeable by changing an amplification ratio of an amplifier of each pixel of the imaging device (21).

9. The endoscope according to any of claims 1 to 8, comprising means for switching between

    (i) a special light observation mode in which the method according to any one of claims 1 to 10 is performed, and
    (ii) a normal observation mode in which brightness levels of the plural captured images are changed at a same ratio.

10. The endoscope according to any of claims 1 to 9, wherein
the first light source section (45) includes
a phosphor (37), and
a semiconductor light emitting element that emits excitation light for the phosphor (37).

11. The endoscope according to any of claims 1 to 9, wherein
the first light source section is adapted to emit light which originates from a xenon light source or a halogen light source.

12. The endoscope according to any one of claims 1 to 11, wherein the second light source section includes a semiconductor light emitting element.

13. The endoscope according to any one of claims 1 to 11, wherein
the second light source section is adapted to generate the narrow-band light by having light originating from a xenon light source or a halogen light source pass through a narrow-band pass filter which only allows to pass light having predetermined narrow-band wavelength components therethrough, and
the second light source section emits the generated narrow-band light.

**Patentansprüche**

1. Endoskop (11), umfassend:

    einen ersten Lichtquellenabschnitt (45), ausgebildet zum Emittieren von weißem Beleuchtungslicht;
    einen zweiten Lichtquellenabschnitt (47), ausgebildet zum Emittieren von schmalbandigem Licht mit einem Wellenlängenband, das schmaler ist als dasjenige des weißen Beleuchtungslichts,
    einen Bildgebungsabschnitt, ausgebildet zum Aufnehmen einer zu betrachtenden Zone mittels eines Bildgebungsgeräts (21), welches Detektorpixel mehrerer Farben aufweist; und
    einen Steuerabschnitt (73), ausgebildet zum Ausführen von folgenden Operationen:

    Veranlassen des Bildgebungsabschnitts, ein Aufnahmebildsignal auszugeben, welches eine Rücklichtkomponente des weißen Beleuchtungslichts enthält, von der zu betrachtenden Zone, und eine Rücklichtkomponente des schmalbandigen Lichts von der zu betrachtenden Zone enthält;
    Erzeugen aufgenommener Bilder mehrerer Referenzfarben basierend auf dem Aufnahmebildsignal, wobei
    ein erstes aufgenommenes Bild der Aufnahmebilder die Rücklichtkomponente des von der zweiten Lichtquelle emittierten schmalbandigen Lichts enthält; und
    Ändern eines Helligkeitspegels des ersten aufgenommenen Bilds unabhängig von dem Helligkeitspegel der übrigen aufgenommenen Bilder,
    wobei der Steuerabschnitt (73) ausgebildet ist zum Ausführen einer derartigen Steuerung, dass sowohl der erste Lichtquellenabschnitt (45) als auch der zweite Lichtquellenabschnitt (47) gleichzeitig eingeschaltet werden, **dadurch gekennzeichnet, dass** der Steuerabschnitt (73) weiterhin ausgebildet ist zum Unterteilen des ersten aufgenommenen Bilds und des zweiten aufgenommenen Bilds, das eine unterschiedliche Referenzfarbe gegenüber derjenigen des ersten aufgenommenen Bilds aufweist, in gemeinsame mehrere Bildbereiche;
    Integrieren von Helligkeitswerten in jedem Bildbereich des ersten aufgenommenen Bilds, um einen integrierten Helligkeitswert für jeden Bildbereich des ersten aufgenommenen Bilds zu erhalten;
    Integrieren von Helligkeitswerten in jedem Bildbereich des zweiten aufgenommenen Bilds, um einen integrierten Helligkeitswert für jeden Bildbereich des zweiten aufgenommenen Bilds zu erhalten;
    Gewinnen eines Verhältnisses von (i) dem integrierten Helligkeitswert jedes Bildbereichs des ersten aufgenommenen Bilds und (ii) desjenigen Bildbereichs des zweiten aufgenommenen Bilds, der die gleiche Lagebeziehung zu jedem Bildbereich des ersten aufgenommenen Bilds besitzt;
    Extrahieren von Bildbereichen des ersten und des zweiten aufgenommenen Bilds, deren Verhältnis gleich oder größer als ein Schwellenwert ist, als charakteristische Bildbereiche; und
    selektives Ändern eines Helligkeitspegels des charakteristischen Bildbereichs des ersten aufgenommenen Bilds.

**2.** Endoskop nach Anspruch 1, bei dem eine Mittelwellenlänge des von der zweiten Lichtquelle (47) emittierten schmalbandigen Lichts im Bereich von 360 nm bis 470 nm liegt.

**3.** Endoskop nach einem der Ansprüche 1 und 2, bei dem
das Ändern des Helligkeitspegels ein Ändern einer Korrekturmatrix beinhaltet, die zum Korrigieren von Helligkeitswerten jeweiliger Pixel in dem Aufnahmebildsignal durch eine Matrixoperation verwendet wird,
wobei der Steuerabschnitt weiterhin ausgebildet ist zum Korrigieren eines Aufnahmebildsignals, welches neuerlich von dem Bildgebungsabschnitt gewonnen wird, unter Verwendung der geänderten Korrekturmatrix.

**4.** Endoskop nach Anspruch 3, bei dem der Steuerabschnitt weiterhin ausgebildet ist, um, wenn ein Helligkeitspegel eines Bilds, welches erhalten wurde durch Korrigieren des neuerlich von dem Bildgebungsabschnitt erhaltenen aufgenommenen Bilds unter Verwendung der geänderten Korrekturmatrix, einen vorbestimmten Ziel-Helligkeitspegel übersteigt, die Korrekturmatrix so zurückzusetzen, dass der Helligkeitspegel des korrigierten Bilds verringert wird.

**5.** Endoskop nach einem der Ansprüche 1 bis 4, bei dem
Lichtkomponenten erfasster Farben, die von der Bildgebungseinrichtung erfasst werden, Lichtkomponenten eines Primärfarbsystems mit den Farben Blau, Grün und Rot enthalten,
die Referenzfarbe des ersten aufgenommenen Bilds Blau ist, und
die Referenzfarbe des zweiten aufgenommenen Bilds Grün ist.

**6.** Endoskop nach einem der Ansprüche 1 bis 4, bei dem
Lichtkomponenten der erfassten Farben, die von der Bildgebungseinrichtung erfasst werden, Lichtkomponenten eines Komplementärfarbsystems mit den Farben Magenta, Zyan und Gelb enthalten,
die Lichtkomponenten der jeweiligen Detektorfarben umgewandelt werden in Lichtkomponenten eines Primärfarbsystems mit den Farben Blau, Grün und Rot,
die Referenzfarbe des ersten aufgenommenen Bilds Blau ist,
die Referenzfarbe des zweiten aufgenommenen Bilds Grün ist.

**7.** Endoskop nach einem der Ansprüche 1 bis 6, bei dem die Bildgebungseinrichtung (21) einen CCD-Sensor enthält.

**8.** Endoskop nach einem der Ansprüche 1 bis 6, bei dem
die Bildgebungseinrichtung (21) einen CCD-Bildsensor enthält, und
der Helligkeitspegel des ersten aufgenommenen Bilds änderbar ist durch Ändern eines Verstärkungsverhältnisses eines Verstärkers jedes Pixels der Bildgebungseinrichtung (21).

**9.** Endoskop nach einem der Ansprüche 1 bis 8, umfassend eine Einrichtung zum Umschalten zwischen

(i) einem Speziallicht-Betrachtungsmodus, in welchem das Verfahren der Ansprüche 1 bis 9 ausgeführt wird, und
(ii) einem Normalbetrachtungsmodus, in welchem die Helligkeitspegel der mehreren aufgenommenen Bilder in gleichem Verhältnis geändert werden.

**10.** Endoskop nach einem der Ansprüche 1 bis 9, bei dem
der erste Lichtquellenabschnitt (45) einen Leuchtstoff (37) enthält, und
ferner ein Halbleiter-Lichtemissionselement, welches Anregungslicht für den Leuchtstoff (37) emittiert.

**11.** Endoskop nach einem der Ansprüche 1 bis 9, bei dem
der erste Lichtquellenabschnitt ausgebildet ist zum Emittieren von Licht, welches aus einer Xenon-Lichtquelle oder einer Halogen-Lichtquelle stammt.

**12.** Endoskop nach einem der Ansprüche 1 bis 11, bei dem der zweite Lichtquellenabschnitt ein Halbleiter-Lichtemissionselement enthält.

**13.** Endoskop nach einem der Ansprüche 1 bis 11, bei dem
der zweite Lichtquellenabschnitt ausgebildet ist zum Erzeugen von schmalbandigem Licht, indem Licht aus einen Xenon-Lichtquelle oder einer Halogen-Lichtquelle durch ein schmalbandiges Filter hindurchgelangt, welches nur Licht passieren lässt, das vorbestimmte schmalbandige Wellenlängenkomponenten enthält, und
der zweite Lichtquellenabschnitt das erzeugte schmalbandige Licht emittiert.

**Revendications**

**1.** Endoscope (11), présentant :

une première section de source lumineuse (45) apte à émettre une lumière d'éclairage blanche ;
une seconde section de source lumineuse (47) apte à émettre une lumière à bande étroite présentant une bande de longueurs d'onde plus étroite que celle de la lumière d'éclairage blanche ;

une section d'imagerie apte à capturer une région à observer par un dispositif d'imagerie (21) présentant des pixels de détection de plusieurs couleurs, et

une section de commande (73) apte à exécuter des opérations pour

amener la section d'imagerie à produire un signal d'image capturée incluant une composante de lumière de retour de la lumière d'éclairage blanche à partir de la région à observer et une composante de lumière de retour de la lumière à bande étroite à partir de la région à observer ;

générer des images capturées de plusieurs couleurs de référence sur la base du signal d'image capturée, dans lequel

une première image capturée des images capturées contient principalement la composante de lumière de retour de la lumière à bande étroite émise à partir de la seconde source lumineuse, et

modifier un niveau de luminosité de la première image capturée indépendamment des niveaux de luminosité des autres images capturées, dans lequel

la section de commande (73) est en outre apte à réaliser une commande de telle sorte qu'à la fois la première section de source lumineuse (45) et la seconde section de source lumineuse (47) sont mises sous tension simultanément, et **caractérisé en ce que**

la section de commande (73) est en outre apte à diviser la première image capturée et une seconde image capturée, laquelle présente une couleur de référence différente de celle de la première image capturée, en plusieurs zones d'image communes ;

intégrer des valeurs de luminosité dans chaque zone d'image de la première image capturée afin d'obtenir une valeur de luminosité intégrée de chaque zone d'image de la première image capturée ;

intégrer des valeurs de luminosité dans chaque zone d'image de la seconde image capturée afin d'obtenir une valeur de luminosité intégrée de chaque zone d'image de la seconde image capturée ;

obtenir un rapport entre (i) la valeur de luminosité intégrée de chaque zone d'image de la première image capturée et (ii) la valeur de luminosité intégrée de la zone d'image, présentant une relation de position d'image identique avec chaque zone d'image de la première image capturée, de la seconde image capturée ;

extraire des zones d'image, des première et seconde images capturées, dont le rapport est supérieur ou égal à une valeur de seuil, comme zones d'image caractéristiques, et

modifier de manière sélective un niveau de luminosité de la zone d'image caractéristique de la première image capturée.

2. Endoscope selon la revendication 1, dans lequel une longueur d'onde centrale de la lumière à bande étroite émise à partir de la seconde source lumineuse (47) est comprise dans une plage de 360 nm à 470 nm.

3. Endoscope selon l'une quelconque des revendications 1 à 2, dans lequel
la modification du niveau de luminosité inclut la modification d'une matrice de correction, laquelle est utilisée pour corriger les valeurs de luminosité de pixels respectifs dans le signal d'image capturée par une opération matricielle,
la section de commande est en outre apte à corriger un signal d'image capturée, lequel est nouvellement obtenu à partir de la section d'imagerie, en utilisant la matrice de correction modifiée.

4. Endoscope selon la revendication 3, dans lequel la section de commande est en outre apte
si un niveau de luminosité d'une image obtenue en corrigeant le signal d'image capturée, lequel est nouvellement obtenu à partir de la section d'imagerie, en utilisant la matrice de correction modifiée, dépasse un niveau de luminosité cible prédéterminé, à réinitialiser la matrice de correction de manière à diminuer le niveau de luminosité de l'image corrigée.

5. Endoscope selon l'une quelconque des revendications 1 à 4, dans lequel
les composantes de lumière de couleurs de détection, lesquelles sont détectées par le dispositif d'imagerie, incluent des composantes de lumière d'un système de couleurs primaires contenant le bleu, le vert, et le rouge ;
la couleur de référence de la première image capturée est le bleu, et
la couleur de référence de la seconde image capturée est le vert.

6. Endoscope selon l'une quelconque des revendications 1 à 4, dans lequel
les composantes de lumière de couleurs de détection, lesquelles sont détectées par le dispositif d'imagerie, incluent des composantes de lumière d'un système de couleurs complémentaires contenant le magenta, le cyan, et le jaune ;
les composantes de lumière des couleurs de détection respectives sont converties en composantes de lumière du système de couleurs primaires du bleu, du vert, et du rouge ;

la couleur de référence de la première image capturée est le bleu, et

la couleur de référence de la seconde image capturée est le vert.

7. Endoscope selon l'une quelconque des revendications 1 à 6, dans lequel le dispositif d'imagerie (21) inclut un capteur d'image de type CCD (Charge-Coupled Device, dispositif à couplage de charge).

8. Endoscope selon l'une quelconque des revendications 1 à 6, dans lequel

le dispositif d'imagerie (21) inclut un capteur d'image de type CCD, et

le niveau de luminosité de la première image capturée peut être modifié en modifiant un rapport d'amplification d'un amplificateur de chaque pixel du dispositif d'imagerie (21).

9. Endoscope selon l'une quelconque des revendications 1 à 8, comprenant un moyen pour la commutation entre :

(i) un mode d'observation à lumière spécial, où le procédé selon l'une quelconque des revendications 1 à 10 est exécuté, et

(ii) un mode d'observation normal, où les niveaux de luminosité de la pluralité d'images capturées sont modifiés avec un rapport identique.

10. Endoscope selon l'une quelconque des revendications 1 à 9, dans lequel

la première section de source lumineuse (45) inclut :

un luminophore (37), et

un dispositif électroluminescent à semi-conducteur, lequel émet une lumière d'excitation pour le luminophore (37).

11. Endoscope selon l'une quelconque des revendications 1 à 9, dans lequel

la première section de source lumineuse est apte à émettre une lumière, laquelle provient d'une source lumineuse au xénon ou d'une source lumineuse halogène.

12. Endoscope selon l'une quelconque des revendications 1 à 11, dans lequel la seconde section de source lumineuse inclut un dispositif électroluminescent à semi-conducteur.

13. Endoscope selon l'une quelconque des revendications 1 à 11, dans lequel

la seconde section de source lumineuse est apte à générer la lumière à bande étroite en faisant en sorte qu'une lumière provenant d'une source lumineuse au xénon ou d'une source lumineuse halogène passe à travers un filtre à bande étroite, lequel ne laisse passer que la lumière présentant des composantes de longueurs d'onde à bande étroite prédéterminées, et

la seconde section de source lumineuse émet la lumière à bande étroite générée.

# FIG. 1

*LSCS: light source control section

*1stSPS: 1st signal processing section
*SSS: Signal separating section
*MCS: Matrix calculating section
*2ndSPS: 2nd signal processing section

FIG. 2

FIG. 3

Laser light of 405nm  (A)

Laser light of 445nm

Excited light emitted from phosphor

} White light (B)

Emission light intensity

405 445    500              600              700

Wavelength(nm)

FIG. 4

FIG. 5

FIG. 6

White light observation image

Narrow-band light observation image

# FIG. 7

```
                    ┌─────────────┐
                    │    Start    │
                    └─────────────┘
                           │
  ┌──────────────────────────────────────────────────────┐
  │         Acquire captured images (R, G, B)            │────  S1
  └──────────────────────────────────────────────────────┘
                           │
  ┌──────────────────────────────────────────────────────┐
  │           Divide into plural image areas            │────  S2
  └──────────────────────────────────────────────────────┘
                           │
  ┌──────────────────────────────────────────────────────┐
  │  Obtain corrected image data (Rc, Gc, Bc) by weighting process  │────  S3
  └──────────────────────────────────────────────────────┘
                           │
  ┌──────────────────────────────────────────────────────┐
  │         Calculate reference brightness value A       │────  S4
  └──────────────────────────────────────────────────────┘
                           │
  ┌──────────────────────────────────────────────────────┐
  │  Change brightness levels of captured images so that reference brightness value  │────  S5
  │      A is brought to be close to target brightness value TL    │
  └──────────────────────────────────────────────────────┘
                           │
  ┌──────────────────────────────────────────────────────┐
  │  Obtain integrated brightness values GSij, BSij of image areas Mij for captured  │────  S6
  │                      images (G, B)                   │
  └──────────────────────────────────────────────────────┘
                           │
  ┌──────────────────────────────────────────────────────┐
  │  Extract characteristic image area(s) MC(k) whose brightness ratio GSij/BSij is  │────  S7
  │           larger than reference brightness ratio αc  │
  └──────────────────────────────────────────────────────┘
                           │
  ┌──────────────────────────────────────────────────────┐
  │  Obtain emphasized image data (Be) in which MC(k) are emphasized  │────  S8
  └──────────────────────────────────────────────────────┘
                           │
  ┌──────────────────────────────────────────────────────┐
  │  Obtain integrated brightness value BeS of emphasized image data (Be)  │────  S9
  └──────────────────────────────────────────────────────┘
                           │
  ┌──────────────────────────────────────────────────────┐
  │  Change brightness level of captured image so that BeS is brought to be close to  │────  S10
  │        characteristic image target brightness value TLc        │
  └──────────────────────────────────────────────────────┘
                           │
  ┌──────────────────────────────────────────────────────┐
  │        Acquire new captured images (Ra, Ga, Ba)      │────  S11
  └──────────────────────────────────────────────────────┘
                           │
  ┌──────────────────────────────────────────────────────┐
  │  Change brightness levels of captured images of respective colors at the same  │────  S12
  │  ratio so that reference brightness value A of captured images is brought to be  │
  │             close to target brightness value TL      │
  └──────────────────────────────────────────────────────┘
                           │
                    ┌─────────────┐
                    │     End     │
                    └─────────────┘
```

FIG. 8A

R,G,B

B2

FIG. 8B

Mij

i

j

FIG. 8C

PB

PA

FIG. 8D

Rc,Gc,Bc

B2

FIG. 9A

B,G

MC(1)

MC(2)

MC(3)

FIG. 9B

Be

FIG. 9C

FIG. 9D

# FIG. 10

Intensity

# FIG. 11

From S5

| Obtain ratios of brightness values of captured images (B, G) at the same pixel positions and extract characteristic pixel(s) whose ratio is equal to or larger than predetermined value | S6A |
|---|---|

| Divide into plural image areas Mij, obtain the number of characteristic pixels in each image area Mij, and extract image area(s) whose number of characteristic pixels is equal to or larger than threshold value as characteristic image area MC(k) | S7A |
|---|---|

To S8

FIG. 12

41     25A     11

49     47     85

55B

LSCS

Light source for special light ➝ Narrow-band light

White light source ➝ White illumination light

81     83

*LSCS: Light source control section

EP 2 301 415 B1

# FIG. 13

*LSCS: Light source control section

FIG. 14

*VCC: Vertical scanning circuit

EP 2 301 415 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002034893 A
- US 20030176768 A
- US 20080281154 A
- US 20080294105 A
- EP 2052673 A
- EP 2052673 A1
- US 2007203413 A1